# EUROPÄISCHE PATENTSCHRIFT

(11) **EP 3 250 254 B1**
(45) Veröffentlichungstag und Bekanntmachung des Hinweises auf die Patenterteilung: **18.09.2019**
(21) Anmeldenummer: 16709962.1
(22) Anmeldetag: 26.01.2016
(51) Int. Cl.: A61M 1/36, A61M 25/06, A61M 1/10

(54) **ANORDNUNG MIT EINER SAUGLEITUNG, EINER DRUCKLEITUNG UND EINER PUMPE**
ASSEMBLY COMPRISING A SUCTION LINE, A PRESSURE LINE AND A PUMP
AGENCEMENT COMPORTANT UNE CONDUITE D'ASPIRATION, UNE CONDUITE DE REFOULEMENT ET UNE POMPE

(30) Priorität: 26.01.2015 DE 102015000771
(43) Veröffentlichungstag der Anmeldung: 06.12.2017
(73) Patentinhaber: Xenios AG, 74076 Heilbronn (DE)
(72) Erfinder: SIMUNDIC, Ivo, 73240 Wendlingen (DE); MATHEIS, Georg, 74076 Heilbronn (DE); OSTADAL, Petr, 19000 Prag (CZ)
(74) Vertreter: Castell, Klaus
(86) Internationale Anmeldenummer: PCT/DE2016/000025
(87) Internationale Veröffentlichungsnummer: WO 2016/119771

(56) Entgegenhaltungen:
- US-A- 5 011 469
- US-A1- 2002 087 107
- US-A1- 2008 249 456
- US-A1- 2011 112 353
- US-A1- 2011 160 517

## Beschreibung

Die Erfindung betrifft eine Anordnung mit einer Saugleitung, die eine venöse Kanüle aufweist, und einer Druckleitung, die eine arterielle Kanüle aufweist, und einer Pumpe, die zwischen Saugleitung und Druckleitung angeordnet ist.

Derartige Anordnungen werden für die extrakorporale Membranoxygenierung oder auch extrakorporale Lungenunterstützung verwendet. Für die extrakorporale Membranoxygenierung werden Kanülen in zwei große Blutgefäße eingebracht. Das ECMO-Gerät pumpt Blut durch einen Membranoxygenator, der den Gasaustausch in der Lunge ersetzt. Das so aufbereitete Blut wird dann zum Patienten geführt. Die Erfindung betrifft insbesondere eine Vorrichtung für die Veno-arterielle ECMO (VA-ECMO). Bei der VA-ECMO wird Blut aus großen Venen, wie insbesondere der Vena femoralis, entnommen und am Herzen vorbei in eine Arterie (Arteria femoralis) geleitet, sodass ein Parallelkreislauf entsteht. Da dadurch das Herz entlastet ist, wird diese Methode bei Patienten mit schlechter Pumpfunktion des Herzens (z.B. Herzinsuffizienz, kardiogener Schock) eingesetzt. Derartige Systeme werden auch als extrakorporale Life-Support-Systeme (ECLS) bezeichnet.

Die DE 695 24 217 T2 beschreibt einen Kardioplegiekatheter, der über das Schlüsselbein eingeführt wird und mit dem eine Mischung aus Blut und Kardioplegielösung oder eine reine kristalloide Lösung verabreicht wird. Dieser Katheter hat eine arterielle Kanüle, an die sich eine kurze Entlastungskanüle mit einem Ballon anschließt.

Die DE 89 90 089 U1 beschreibt eine aufwändige Anordnung mit mehreren Pumpen, Entlastungsleitungen und Reservoiren, die in der Praxis schwer zu bedienen ist.

Die US 2002/087107 A1 und die US 2011/112353 beschreiben Systeme, die zur Herzunterstützung während einer Operation am offenen Herzen oder subkutan eingesetzt werden. Der Aufbau und die Anordnung der Katheter ermöglicht es aber nicht, das Herz auf der Seite der gleichen Herzkammer mit einer arteriellen Kanüle zu versorgen und gleichzeitig mit einer Ventkanüle zu entlasten.

Der Erfindung liegt die Aufgabe zu Grunde, ein derartiges ECLS-System weiterzuentwickeln.

Dies wird dadurch erreicht, dass die gattungsgemäße Anordnung eine Entlastungsleitung mit einer Entlastungskanüle aufweist, wobei die Entlastungskanüle länger ist als die arterielle Kanüle und die Entlastungsleitung mit der Saugleitung oder der Druckleitung verbunden ist. Insbesondere ist Entlastungskanüle als Ventkanüle ausgebildet, die länger ist als die arterielle Kanüle, und die Entlastungsleitung ist direkt mit der Saugleitung oder direkt mit der Druckleitung verbunden, wobei zwischen Saugleitung und Druckleitung nur eine einzige Pumpe und kein Reservoir angeordnet ist.

Im Gegensatz zur DE 695 24 217 T2 ist die erfindungsgemäße Entlastungskanüle vorzugsweise eine einstückige durchgehende Kanüle. Die erfindungsgemäße Kanüle hat keinen Ballon an dem in das Herz einführbaren Ende. Das proximale Ende ist im Gegensatz zu einem Ballonkatheter erfindungsgemäß gleichbleibend im Durchmesser, so dass neben der Ventkanüle Blut strömen kann. Somit hat die erfindungsgemäße Ventkanüle auch keinen weiteren Zugang zu einem Ballon. Dadurch kann der an der Kanüle zur Verfügung stehende Durchmesser vollständig für einen Blutstrom genutzt werden.

Die erfindungsgemäße Kanüle hat somit nur ein Lumen für einen Blutstrom in einer Richtung hin zum Herz und ein Lumen für einen Blutstrom weg vom Herz. Weitere Lumen oder Kanäle werden nicht benötigt und sind sogar von Nachteil.

Außerdem ist bei der DE 695 24 217 T2 am proximalen flexiblen Ende ein Sensor vorgesehen. Auch der Sensor führt zu einem aufwändigeren Aufbau und erfordert eine Leitung in der Kanüle, um die gemessenen Werte weiterzuleiten. Die erfindungsgemäße Anordnung besteht somit ausschließlich aus Blutleitungen und sie benötigt weder elektrische Leitungen zum Weiterleiten von Sensorsignalen noch pneumatische Leitungen zum Bedienen eines Ballons.

Die DE 89 90 089 U1 beschreibt wie die DE 695 24 217 T2 ein Bypass-System, bei dem das Herz zuerst blutleer gepumpt wird. Daher ist bei derartigen Systemen ein Reservoir als Kardiotomiereservoir notwendig. Die erfindungsgemäße Anordnung ist im Gegensatz hierzu für die Therapie bestimmt und benötigt kein derartiges Kardiotomiereservoir.

Die Entlastungskanüle ermöglicht es, einen Entlastungsblutstrom aus dem Herzen zurückzuführen, um das Herz im Moment des Zustroms durch die arterielle Kanüle zu entlasten. Die arterielle Kanüle endet dabei vorzugsweise bereits vor dem Herzen in der Arterie, während die Entlastungskanüle bis weit ins Herz hineingeschoben wird. Die Entlastungskanüle ist daher in der Praxis mindestens 20 % länger als die arterielle Kanüle.

Die Anordnung kann zur reinen Herzunterstützung eingesetzt werden. Erfindungsgemäß ist jedoch ein Einsatz als Lungenunterstützung, bei dem ein Oxygenator zwischen Saugleitung und Druckleitung angeordnet ist.

Besonders vorteilhaft ist die Entlastungsleitung, wenn die Pumpe einen pulsierenden Durchfluss erzeugt. Dann kann im Moment der Druckerhöhung an der Pumpe und damit in der Arterie und im Herzen eine Entlastung durch die Entlastungsleitung erreicht werden. Die Anordnung eignet sich daher vor allem für pulsierende Pumpen. Insbesondere im Rahmen eines pulsatilen Betriebs zur Kreislaufunterstützung ist ein Oxygenator notwendig, da das Blut teilweise an den Lungen vorbei geleitet wird.

Um unabhängig vom Durchmesser der Entlastungsleitung die Intensität der Entlastung einstellen zu können, wird vorgeschlagen, dass die Entlastungsleitung einen Durchflussbegrenzer aufweist. Dieser Durchflussbegrenzer kann den durch die Entlastungsleitung geführten Volumenstrom reduzieren, um eine stärkere oder geringere Entlastung am Herzen einstellen zu können.

Besonders vorteilhaft ist eine Steuerung oder eine Regelung, die es ermöglicht, den Durchflussbegrenzer in Abhängigkeit vom pulsierenden Durchfluss automatisch einzustellen. Dies ermöglicht es, bei einer Druckerhöhung der pulsierenden Pumpe eine Entlastung herbeizuführen und den Moment der Entlastung in Abhängigkeit vom Moment der Druckerhöhung und insbesondere in Abhängigkeit vom Pumprhythmus des Herzens zu steuern oder zu regeln.

Um den Rückstrom der Entlastungsleitung mit möglichst wenigen Turbulenzen in die Saugleitung oder in die Druckleitung einzuleiten, wird vorgeschlagen, dass zwischen der Entlastungsleitung und der Saugleitung oder der Druckleitung ein Y-Adapter angeordnet ist. Dieser Y-Adapter ist jeweils so angeordnet, dass der zuströmende Blutfluss sich in einem stumpfen Winkel mit dem jeweils anderen Blutfluss vereinigt.

Allein die Verbindung der Entlastungsleitung mit der Druck- oder Saugleitung über einen Y-Adapter sorgt dafür, dass die Entlastungskanüle im Herzen eine Entlastung durch Ableiten von Blut bewirkt. Sofern die Entlastungsleitung mit der Druckleitung verbunden ist, ist es jedoch in vielen Fällen vorteilhaft, wenn die Entlastungsleitung ein Rückschlagventil aufweist. Dies verhindert, dass die Pumpe Blut in die Entlastungsleitung pumpt.

Eine besondere Ausführungsform sieht vor, dass die Entlastungsleitung über eine Venturidüse mit der Druckleitung verbunden ist. Über eine Venturi- oder Injektordüse wird somit im Bereich der Einmündung der Entlastungsleitung ein Unterdruck erzeugt, der für eine Absaugung von Blut aus der Entlastungsleitung sorgt.

Für die der Erfindung zu Grunde liegende Aufgabe eignet sich vor allem eine Kanüle, die 80 cm bis 100 cm, vorzugsweise zwischen 85 cm und 95 cm lang ist. Vorteilhaft ist eine Größe von 7 Fr bis 9 Fr oder 2 mm bis 3 mm Außendurchmesser.

Vorteilhaft ist es, wenn die Kanüle einen Durchflussbegrenzer aufweist. Hierunter wird auch eine Kanüle verstanden, die über eine Leitung mit einem Durchflussbegrenzer in Verbindung steht. Ein derartiger Durchflussbegrenzer sollte möglichst automatisch einstellbar sein, um an den Puls der Pumpe und vorzugsweise auch an den Puls des Herzens angepasst zu werden.

Mehrere Ausführungsbeispiele erfindungsgemäßer Anordnungen sind in der Zeichnung dargestellt und werden im Folgenden näher erläutert. Es zeigt
- Figur 1: ein extrakorporales Life-Support-System mit in die Saugleitung führender Entlastungsleitung,
- Figur 2: ein extrakorporales Life-Support-System mit in die Druckleitung führender Entlastungsleitung,
- Figur 3: einen Ausschnitt des extrakorporalen Life-Support-Systems gemäß Figur 1 mit einer zweiten Pumpe,
- Figur 4: ein extrakorporales Life-Support-System gemäß Figur 2 mit einer zweiten Pumpe.
- Figur 5: eine Anordnung gemäß Figur 4 mit einem Y-Adapter,
- Figur 6: die Führung einer Druckleitung und einer kleineren Entlastungsleitung in einer Kanüle,
- Figur 7: einen Querschnitt einer Führung einer Entlastungsleitung in einer Kanülenwand,
- Figur 8: einen Längsschnitt zu der in Figur 7 gezeigten Kanülenführung,
- Figur 9: schematisch einen bilateralen Zugang der Entlastungskanüle,
- Figur 10: schematisch einen Zugang der Entlastungskanüle durch eine Ellenbeugervene,
- Figur 11: schematisch einen Zugang der Entlastungskanüle über die obere Hohlvene und ein Vorhofseptum und
- Figur 12: schematisch einen Zugang der Entlastungskanüle über die untere Hohlvene und ein Vorhofseptum.

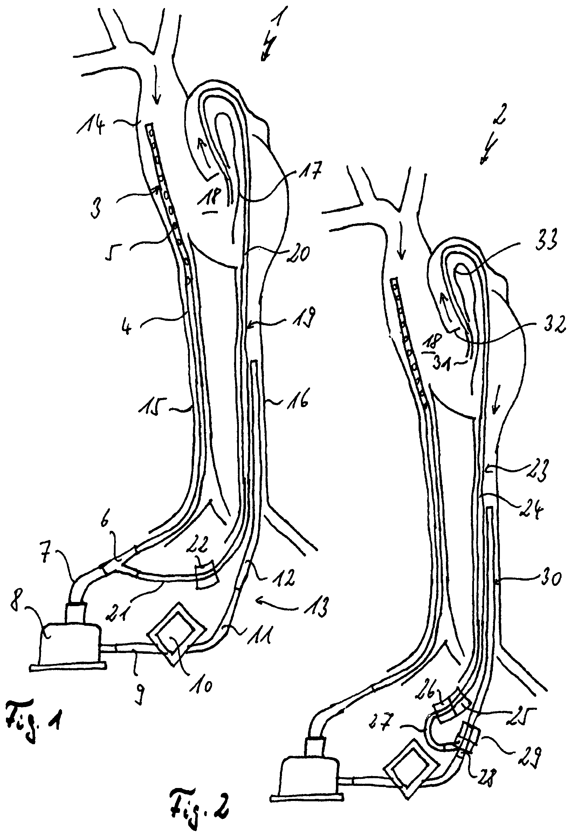

Das in den Figuren gezeigte extrakorporale Life-Support-System 1, 2 weist im ersten Ausführungsbeispiel der Figur 1 eine Saugleitung 3 auf, die eine venöse Kanüle 4 mit Bohrungen 5, einen Y-Adapter 6 und eine Zuleitung 7 zu einer Pumpe 8 aufweist. Von der Pumpe 8 ist nur der Pumpenkopf gezeigt. Die Pumpe 8 ist über eine Verbindungsleitung 9 mit einem Oxygenator 10 verbunden, der über eine Ableitung 11 mit einer arteriellen Kanüle 12 verbunden ist. Die Ableitung 11 und die arterielle Kanüle 12 bilden eine Druckleitung 13.

Im Einsatz der Anordnung kann somit über die venöse Kanüle 4 der Saugleitung 3 aus einem Herz 14 über die Vena femoralis 15 Blut zur Pumpe 8 gezogen werden, um anschließend über den Oxygenator 10 und die arterielle Kanüle 12 über die Arteria femoralis 16 und die Aorta über den Aortenbogen in den linken Ventrikel geführt werden. Dadurch entsteht ein Bypass zum Herzen 14, der das Herz entlastet.

Bei einer pulsierenden Pumpe 8 entsteht im Moment des höchsten Druckes ein Überdruck in der Arteria femoralis 16, der auf die Herzwand 17 drückt. Daher ist es vorteilhaft, wenn in diesem Moment im Bereich 18 hinter der Herzwand 17 durch Absaugen von Blut der Druck vermindert wird. Dies wird durch die Entlastungsleitung 19 erreicht, die eine Entlastungskanüle 20 und eine Entlastungsleitung 21 aufweist. Diese Entlastungskanüle 20 erlaubt es, Blut aus dem Herzen 14 durch die Arteria femoralis 16 zum Y-Adapter 6 zu führen, von wo das Blut über die Zuleitung 7 zur Pumpe 8 gelangt. Die Pumpe 8 saugt somit nicht nur Blut aus der venösen Kanüle 4 sondern auch aus der Entlastungskanüle 20. Auch ohne eine Saugwirkung dient die Entlastungskanüle bereits einem Abbau eines Überdruckes und somit einer Entlastung des Herzens.

Der über die Entlastungsleitung 19 zurückfließende Volumenstrom kann über den Durchflussbegrenzer 22 variiert werden. Der Durchflussbegrenzer 22, der optional vorgesehen werden kann, steht mit einer Steuerung (nicht gezeigt) in Verbindung, die den Durchfluss in der Entlastungsleitung 19 und die Pumpe 8 steuert oder regelt. Dadurch kann die Entlastung während des Betriebs der Pumpe im Einsatz der Anordnung beliebig variiert werden und insbesondere in Abhängigkeit von der Pumpenleistung gesteuert werden. Eine besondere Ausführungsvariante sieht vor, dass die Pumpenleistung und damit indirekt auch der Durchflussbegrenzer in Abhängigkeit vom Herzrhythmus, das heißt dem EKG-Signal, gesteuert werden.

Die in Figur 2 gezeigte alternative Ausführungsform einer Anordnung 2 ist im Wesentlichen wie die in Figur 1 gezeigte Anordnung aufgebaut und eingesetzt. Die Entlastungsleitung 23 weist jedoch eine Entlastungskanüle 24 auf, die über einen Durchflussbegrenzer 25, ein Rückschlagventil 26 und eine Leitung 27 mit einem Y-Adapter 28 in Verbindung steht. Der Y-Adapter 28 ist im vorliegenden Ausführungsbeispiel als Venturidüse 29 ausgebildet. Dies ermöglicht auch einen einfacheren Aufbau ohne Rückschlagventil 26 und ohne Durchflussbegrenzer 25, da die Venturidüse im Moment eines erhöhten Durchflusses in der Druckleitung 30 auch einen stärkeren Unterdruck und damit einen stärkeren Sog an der Entlastungsleitung 23 bewirkt.

Wie bei dem in Figur 1 gezeigten Ausführungsbeispiel liegt bei der Verwendung der Kanüle der Kanüleneinlass 31 der Entlastungsleitung 23 der Entlastungskanüle 24 im Bereich 18 hinter der Aortenklappe 32 und dem Aortenbogen 33.

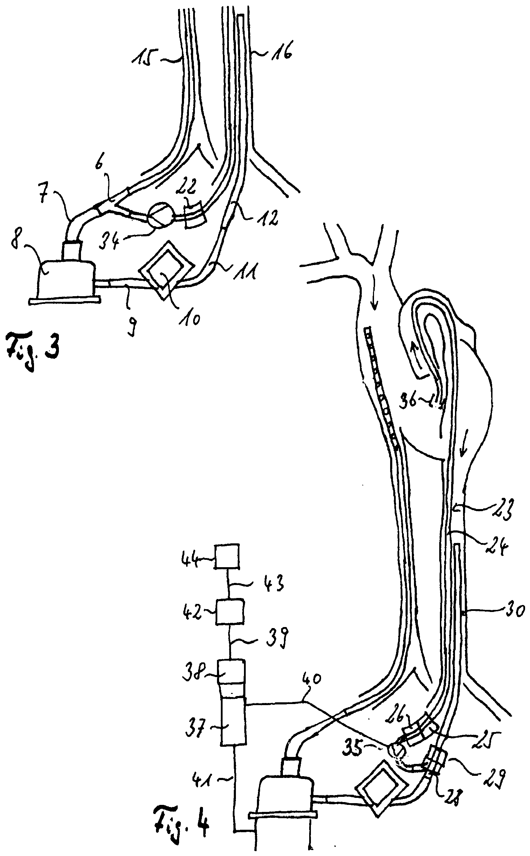

Die Figur 3 zeigt eine Ausführungsform, die im Wesentlichen wie die in Figur 1 gezeigte Ausführungsform aufgebaut ist. Bei diesem Ausführungsbeispiel ist aber eine Pumpe 34, die vorzugsweise als Saugpumpe ausgebildet ist, zwischen der Entlastungsleitung 19 und dem Y-Adapter 6 vorgesehen. Diese Pumpe 34 kann als Saugpumpe unabhängig von der Pumpe 8 angesteuert werden. Sie kann nicht pulsatil oder pulsatil betrieben und synchron mit der Pumpe 8 oder phasenverschoben zur Pumpe 8 betrieben werden. Dabei übernimmt die Pumpe 8 die Funktion der Hauptpumpe und die Pumpe 34 übernimmt die Funktion einer Unterstützungspumpe.

In der Figur 4, die ein Ausführungsbeispiel gemäß Figur 2 zeigt, ist ebenfalls eine weitere als Pumpe 35 zwischen der Entlastungsleitung 23 und dem Y-Adapter 28 vorgesehen. Auch bei diesem Ausführungsbeispiel ist eine EKG getriggerte pulsatile Steuerung mit oder ohne weitere Saugpumpe 35 vorteilhaft.

Hierfür wird eine Pumpensteuerung 37 vorgesehen, die mit der Pumpe 8 und - sofern vorhanden - mit einer weiteren Pumpe 35 (vgl. Figur 4) oder einer weiteren Pumpe 34 (vgl. Figur 3) in Verbindung steht. Ein Rechner 38 wandelt ein Steuersignal 39 in ein Pumpenantriebssignal 40, 41. Dieses Pumpenantriebssignal bewirkt über die Pumpensteuerung 37 eine wellenartig an- und abschwellende Pumpenleistung an der Pumpe 8 und kann darüber hinaus auch für eine synchrone oder zeitversetzte, pulsatile oder nicht pulsatile, von der Hauptpumpe 8 abhängige oder unabhängige Pumpenleistung an den Pumpen 34 oder 35 sorgen. Das Steuersignal 39 wird von einem EKG 42 bereitgestellt, das über ein Kabel 43 mit einem Patienten 44 in Verbindung steht.

Beim Betrieb des ECLS-Systems wird mit dem EKG 42 über das Kabel 43 ein EKG Signal eines Patienten 44 erfasst, um das Steuersignal 39 zu erzeugen. Dieses Steuersignal 39 wird über den Rechner 38 in das Pumpensignal 40, 41 umgewandelt, das über die Pumpensteuerung 37 die Pumpen 8, 34 und 35 steuert oder mit einem Strom versorgt. Dies ermöglicht es, einen SW-Trigger zum Betrieb der Pumpen nach einem speziellen Algorithmus mit dem Ziel abzugeben, Impulse in die Systole und/oder die Diastole abzugeben. Eine derartige Vorrichtung und ein derartiges Verfahren sind in der EP 2 832 383 beschrieben.

Bei dem in der Figur 4 gezeigten Ausführungsbeispiel ist am Ende 31 der Entlastungskanüle 24 ein Abstandshalter 36 vorgesehen. Dieser Abstandshalter 36 verhindert das Ansaugen des Kanüleneinlasses 31 an der Herzwand 17. Die kann durch eine käfigartige Ausbildung oder eine spiralförmige Ausbildung des Endes 31 erreicht werden, die als Pigtail bekannt ist.

In allen Ausführungsbeispielen ist die venöse Kanüle 4 55 cm lang und hat eine Größe von vorzugsweise 19 Fr bis25 Fr. Beispielweise hat die Kanüle eine Größe von 21 bis 25 Fr. Die arterielle Kanüle hat eine Länge von vorzugsweise 38 cm und eine Größe von 13 Fr bis 17 Fr und vorzugsweise von 15 bis 16 Fr. Die Entlastungskanüle ist kleiner als die venöse und kleiner als die arterielle Kanüle. Sie hat eine Größe von 7 Fr bis 9 Fr und eine Länge von 90 cm.

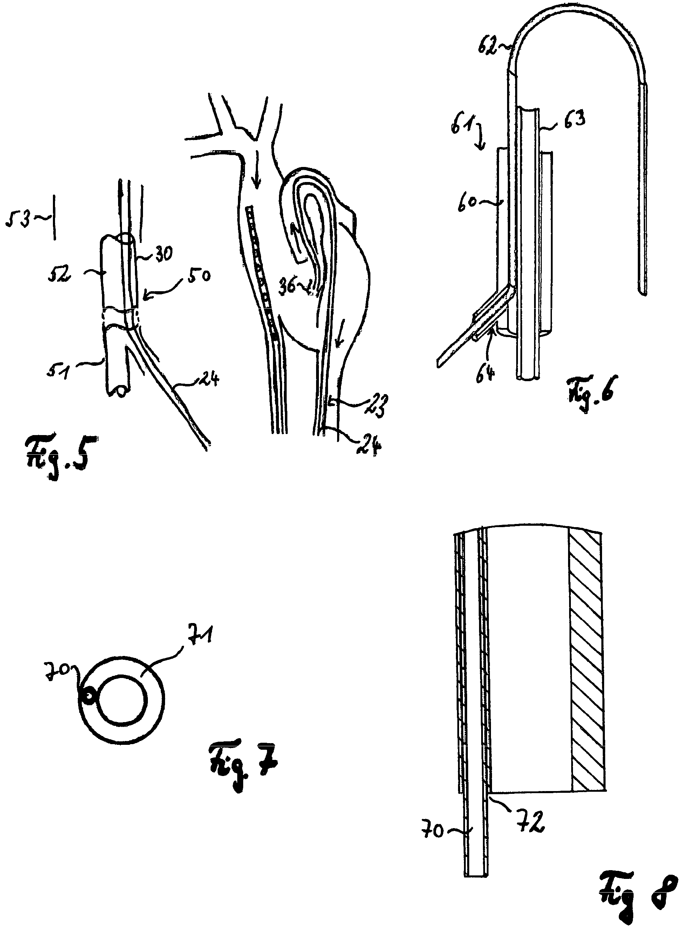

Die Figur 5 zeigt in der leicht vergrößerten Darstellung eines Y-Adapters 50, dass die Entlastungskanüle 24 als Ventkanüle in die Druckleitung 30 eingeführt werden kann, damit nicht zwei Kanülen nebeneinander im Gefäß geführt werden müssen. Die Ventkanüle ist mit 6, 7 oder 8 Fr. ausgebildet und der daneben liegende Schenkel 51 hat 3/8". Der in der angedeuteten Aorta 53 geführte Kanülenschaft 52 der Perfusionskanüle hat 13, 16 oder 18 Fr. und in ihm ist die Ventkanüle 24 flottierend eingeführt.

Bei der in Figur 6 gezeigten Ausführungsform sind innerhalb der Wand 60 eines Katheters 61 die Ventkanüle 62 und die Druckleitung 63 geführt. Dazu ist die Ventkanüle 62 an dem Y-Zugang 64 eingeführt und sie wird dann neben der Druckleitung 63 geführt. Eine nicht gezeigte Ausführungsform sieht vor, die Ventkanüle 62 in die Druckleitung 63 und dann innerhalb der Druckleitung 63 zu führen.

Die Figuren 7 und 8 zeigen, wie eine Ventkanüle 70 in einem Kanüleninnenlumen 71geführt werden kann. Hierfür ist für die Ventkanüle 70 im Lumen 71 ein Arbeitskanal 72 vorgesehen. Im Bereich des Arbeitskanals 72 kann die Wandung der Kanüle verdickt sein, sofern die Wandung nicht dick genug ist, um in ihr einen Arbeitskanal anzuordnen.

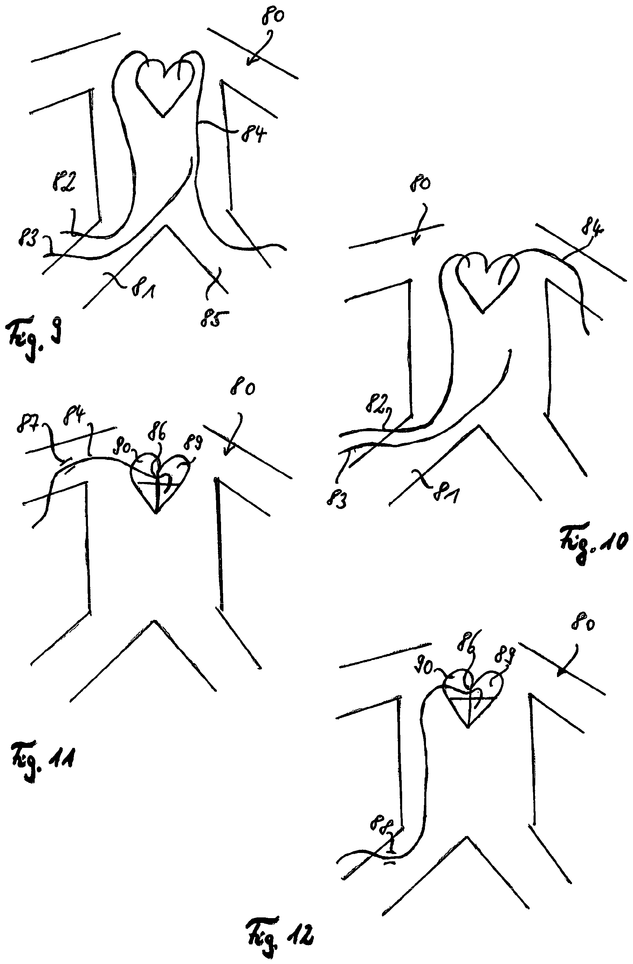

Die Figuren 9 bis 12 zeigen, wie eine Entlastungs- oder Ventkanüle im Körper 80 eines Menschen geführt werden kann. Je nach Führung ergeben sich unterschiedliche Längen und Ausbildungsvarianten der Kanüle.

Bei dem in Figur 9 gezeigten Beispiel wird an einem Bein 81 venös die Saugleitung 82 eingeführt und arteriell die Druckleitung 83. Die Entlastungsleitung 84 wird im anderen Beim 85 verlegt.

In dem in Figur 10 gezeigten Beispiel wird an einem Bein 81 venös die Saugleitung 82 eingeführt und arteriell die Druckleitung 83. Die Entlastungsleitung 84 wird in der Arteria Brachialis (Ellenbeugervene) verlegt.

Die Figuren 11 und 12 zeigen einen Zugang über ein Vorhofseptum 86. Dabei wird die Ventkanüle 84 entweder wie in Figur 11 gezeigt über die obere Hohlvene 87 oder wie in Figur 12 gezeigt über die untere Hohlvene 88 zugeführt. Das Vorhofseptum 86 liegt zwischen den Vorhöfen 89 und 90.

Die gezeigten Ausführungsformen entlasten das Herz insbesondere bei mangelnder Pumpleistung, bzw. Auswurfleistungsfähigkeit. Insbesondere im pulsatilen EGK-getriggerten Betrieb einer oder beider Pumpen verstärkt sich die myokardprotektive Wirkung der diastolischen Augmentation (geringere Nachlast, Erhöhung der linksventrikulären Auswurfleistung, Erniedrigung des linksventrikulären Residualvolumens) durch die Verringerung des Ventrikelvolumens erheblich. Dies führt zu einer weiteren Entlastung des linken Ventrikels und senkt die Wandspannung speziell während der Diastole, wodurch der koronare Fluss positiv beeinflusst werden kann.

## Patentansprüche

1. Anordnung für ein extrakorporales Life-Support-System mit einer Saugleitung (3), die eine venöse Kanüle (4) aufweist, einer Druckleitung (13), die eine arterielle Kanüle (12) aufweist, einer Pumpe (8), die zwischen Saugleitung (3) und Druckleitung (13, 30) angeordnet ist, und einer Entlastungsleitung (19, 23) mit einer Entlastungskanüle (20), wobei die Entlastungskanüle (20) als Ventkanüle ausgebildet ist, die Entlastungsleitung (19) direkt mit der Saugleitung (3) oder direkt mit der Druckleitung (13) verbunden ist und wobei zwischen Saugleitung und Druckleitung nur eine einzige Pumpe und kein Reservoir angeordnet ist, und ein Oxygenator (10) zwischen Saugleitung (3) und Druckleitung (13) angeordnet ist, ***dadurch gekennzeichnet, dass*** die Entlastungskanüle (20) länger ist als die arterielle Kanüle (12).

2. Anordnung nach einem der vorhergehenden Ansprüche, ***dadurch gekennzeichnet, dass*** die Pumpe (8) einen pulsierenden Durchfluss erzeugt.

3. Anordnung nach einem der vorhergehenden Ansprüche, ***dadurch gekennzeichnet, dass*** die Entlastungsleitung (19) einen Durchflussbegrenzer (22) aufweist.

4. Anordnung nach Anspruch 2 und 3, ***dadurch gekennzeichnet, dass*** der Durchflussbegrenzer (22, 25) in Abhängigkeit vom pulsierenden Durchfluss automatisch einstellbar ist.

5. Anordnung nach einem der vorhergehenden Ansprüche, ***dadurch gekennzeichnet, dass*** zwischen der Entlastungsleitung (19) und der Saugleitung (3) oder der Druckleitung (13) ein Y-Adapter (6, 28) angeordnet ist.

6. Anordnung nach einem der vorhergehenden Ansprüche, ***dadurch gekennzeichnet, dass*** die Entlastungsleitung (19) mit der Druckleitung (13) verbunden ist und ein Rückschlagventil (26) aufweist.

7. Anordnung nach einem der vorhergehenden Ansprüche, ***dadurch gekennzeichnet, dass*** die Entlastungsleitung (19) über eine Venturidüse (29) mit der Druckleitung (13) verbunden ist.

## Claims

1. A device for an extracorporeal life support system with a suction line (3) which comprises a venous cannula (4), a pressure line (13) which comprises an arterial cannula (12), a pump (8), which is arranged between the suction line (3) and the pressure line (13, 30) and a relieving line (19, 23) with a relieving cannula (20), wherein the relieving cannula (20) is designed as a vent cannula, the relieving line (19) is directly connected to the suction line (3) or directly to the pressure line (13) and wherein between the suction line and pressure line only a single pump and no reservoir is arranged, and an oxygenator (10) is arranged between the suction line (3) and the pressure line (13), ***characterised in that*** the relieving cannula (20) is longer than the arterial cannula (12).

2. The device according to any one of the preceding claims, ***characterised in that*** the pump (8) produces a pulsating throughflow.

3. The device according to any one of the preceding claims, ***characterised in that*** the relieving line (19) comprises a flow limiter (22).

4. The device according to claim 2 and 3, ***characterised in that*** the flow limiter (22, 25) is automatically adjustable as a function of the pulsating throughflow.

5. The device according to any one of the preceding claims, ***characterised in that*** arranged between the relieving line (19) and the suction line (3) or the pressure line (13) is a Y-adapter (6, 28).

6. The device according to any one of the preceding claims, ***characterised in that*** the relieving line (19) is connected to the pressure line (13) and comprises a non-return valve (26).

7. The device according to any one of the preceding claims, ***characterised in that*** the relieving line (19) is connected to the pressure line (13) via a Venturi nozzle (29).

## Revendications

1. Agencement pour un système extracorporel de maintien des fonctions vitales avec une conduite d'aspiration (3) qui présente une canule veineuse (4), une conduite de refoulement (13) qui présente une canule artérielle (12), une pompe (8) disposée entre la conduite d'aspiration (3) et la conduite de refoulement (13, 30), et une conduite de décharge (19, 23) avec une canule de décharge (20), dans lequel la canule de décharge (20) est réalisée en tant que canule de purge, la conduite de décharge (19) est directement reliée à la conduite d'aspiration (3) ou directement reliée à la conduite de refoulement (13) et dans lequel, entre la conduite d'aspiration et la conduite de décharge seulement une seule pompe est disposée et aucun réservoir, et un oxygénateur (10) est disposé entre la conduite d'aspiration (3) et la conduite de refoulement (13), ***caractérisé en ce que*** la canule de décharge (20) est plus longue que la canule artérielle (12).

2. Agencement selon l'une des revendications précédentes, ***caractérisé en ce que*** la pompe (8) génère un débit pulsé.

3. Agencement selon l'une des revendications précédentes, ***caractérisé en ce que*** la conduite de décharge (19) présente un limiteur de débit (22).

4. Agencement selon la revendication 2 et 3, ***caractérisé en ce que*** le limiteur de débit (22, 25) est réglable automatiquement en fonction du débit pulsé.

5. Agencement selon l'une des revendications précédentes, ***caractérisé en ce que,*** entre la conduite de décharge (19) et la conduite d'aspiration (3) ou la conduite de refoulement (13), un adaptateur Y (6, 28) est disposé.

6. Agencement selon l'une des revendications précédentes, ***caractérisé en ce que*** la conduite de décharge (19) est reliée à la conduite de refoulement (13) et présente une soupape anti-retour (26).

7. Agencement selon l'une des revendications précédentes, ***caractérisé en ce que*** la conduite de décharge (19) est reliée via une buse de Venturi (29) à la conduite de refoulement (13).
